# EUROPÄISCHE PATENTSCHRIFT

(11) **EP 1 033 993 B1**
(45) Veröffentlichungstag und Bekanntmachung des Hinweises auf die Patenterteilung: **10.10.2001**
(21) Anmeldenummer: 98962353.3
(22) Anmeldetag: 18.11.1998
(51) Int. Cl.: A61K 35/74

(54) **VERWENDUNG VON ESCHERICHIA COLI STAMM DSM 6601 ZUR BEHANDLUNG VON DIARRHOEEN AUF DEM VETERINÄRSEKTOR**
USE OF THE E.COLI STRAIN DSM 6601 FOR TREATING DIARRHOEA IN VETERINARY MEDICINE
UTILISATION DE LA SOUCHE E. COLI DSM 6601 POUR TRAITER DES DIARRHEES EN MEDECINE VETERINAIRE

(30) Priorität: 22.11.1997 DE 19751907
(43) Veröffentlichungstag der Anmeldung: 13.09.2000
(73) Patentinhaber: PHARMA-ZENTRALE GMBH, 58313 Herdecke (DE)
(72) Erfinder: PROPPERT, Hans, D-58095 Hagen (DE)
(74) Vertreter: Siewers, Gescha, Dr.
(86) Internationale Anmeldenummer: EP9807389
(87) Internationale Veröffentlichungsnummer: WO9926642

(56) Entgegenhaltungen:
- WO-A-96/13271
- WO-A-98/26787
- DE-A- 19 637 936
- LODINOVA-ZADNIKOVA R AND SONNENBORN U: "Effect of preventive administration of a nonpathogenic Escherichia coli strain on the colonization of the intestine with microbial pathogens in newborn infants" BIOLOGY OF THE NEONATE, Bd. 71, 1997, Seiten 224-232, XP002097854
- KRUIS W ET AL.: "Einsatz von Probiotika in der Humanmedizin" DIE MEDIZINISCHE WELT,1996, Seiten 53-57, XP002097855
- BLUM ET AL: "Properties of Escherichia coli strains of serotype O6" PLASMID, Bd. 23, Nr. 4, Juli 1995, Seiten 234-236, XP002085750
- GEORG K J AND SCHLORER E: "Probiotische Therapie einer pseudomembranosen Kolitis. Kombination aus intestinaler Lavage und oraler Gabe von Escherichia coli" DEUTSCHE MEDIZINISCHE WOCHENSCHRIFT, Bd. 123, Nr. 43, 1998, Seiten 1274-1284, XP002097856

## Beschreibung

Die Erfindung betrifft die Verwendung von Escherichia coli Stamm DSM 6601 zur Prophylaxe und Behandlung von im wesentlichen durch pathogene Pilze bedingten Diarrhoeen bei Tieren.

Unter Diarrhoeen bei Menschen oder Tieren wird die häufige Entleerung von dünnflüssigen Faeces verstanden, deren Ursache unterschiedlich sein kann wie beispielsweise eine allergische Reaktion auf bestimmte Nahrungsmittel, meist aber eine Reaktion auf Mikroorganismen, und zwar Bakterien, Pilze oder Viren oder deren Toxine. Länger dauernde Diarrhoeen können aufgrund des starken Wasser- und Salzverlustes zu schweren Komplikationen bis hin zum Exitus führen. Schwerere Diarrhoeen sind daher auf jeden Fall behandlungsbedürftig, insbesondere dann, wenn es sich um junge oder bereits geschwächte Menschen oder Tiere handelt.

In einem Großteil der Fälle werden Diarrhoeen durch Bakterien verursacht, die nicht zu der normalen Mikroorganismenflora des betreffenden Körpers gehören, wobei insbesondere bestimmte pathogene Stämme von Escherichia coli, Salmonellen und Shigellen eine Hauptrolle spielen. Neben Bakterien können Diarrhoeen aber auch ausgelöst werden durch Infektionen mit Viren, insbesondere Corona- und Rotaviren oder durch Pilze, die in den meisten Fällen zu den Eumykota gerechnet werden. Während bakteriell bedingte Diarrhoeen heute in der Regel durch die Gabe von Sorbentien oder nichtsystemischen Sulfonamiden oder Antibiotika recht gut bekämpft werden können, gibt es noch keine Arzneimittel, die erfolgreich bei Virusinfektionen eingesetzt werden können und kaum Arzneimittel, die bei der Besiedlung des Magen-Darmtraktes mit Pilzen wirksam sind.

Als Pilze bezeichnet man eine polyphylogenetische Gruppe von chlorophyllfreien, heterotrophen und eukaryotischen Organismen, die ein- oder vielzellig sein können und deren Vermehrung wie bei allen Eukaryonten mit Mitose und Meiose verläuft und durch die Bildung geschlechtlicher oder ungeschlechtlicher Sporen, aber auch durch Sprossen. Die einzelligen, sich nur durch Sprossen fortpflanzenden Pilze werden häufig als Hefen zusammengefaßt, obgleich die Hefen ein Konglomerat verschiedener Verwandschaftskreise darstellen. Fast alle Eumykota, also die echten Pilze, durchlaufen während der Individualentwicklung zwei oder mehrere morphologisch unterscheidbare Stadien, nämlich als Teleomorphe, bei der die Sporen nach der Meiose gebildet werden und ein oder mehrere Stadien als Anamorphe, bei denen die Sporenbildung nicht in Verbindung mit der Meiose steht. Pilze, deren Teleomorphe unbekannt sind oder die die Fähigkeit verloren haben, solche zu bilden, werden als Fungi imperfecti zusammengefaßt. Die genaue botanische Klassifikation ist bei den Pilzen häufig noch offen.

Aufgrund ihres Chlorophyllmangels ernähren sich alle Pilze heterotroph durch Abbau organischer Substanzen; sie sind also Saprophyten. Medizinisch unterscheidet man zwischen den Opportunisten, also Saprophyten, die selten und nur unter ganz bestimmten Bedingungen pathogen werden können und wozu beispielsweise Candida und Aspergillus gehören. Sogenannte pathogene Saprophyten führen exogen ein normales saprophytierendes Leben, aber sie sind für Menschen oder Tiere unter allen Umständen als pathogen zu betrachten, wenn eine Infektion stattgefunden hat. Es gibt auch unter den Pilzen obligate Parasiten, die außerhalb des Wirtsorganismus nicht vegetieren können und die nur beim Menschen oder beim Tier gefunden werden; hierzu gehören die meisten Dermatophyten.

Ein wesentlicher Unterschied zwischen Bakterien und Pilzen liegt in der Tatsache begründet, daß Pilze im Gegensatz zu Bakterien Eukaryonten sind, also über einen Kern mit Membran und außerdem im Gegensatz zu Bakterien auch über Mitochondrien verfügen und daß die Zellwand aus Chitin und/oder Cellulose besteht, während die Zellwand der Bakterien aus Mucopeptiden aufgebaut ist. Dies erklärt auch, warum die meisten bei Bakterien wirksamen Antibiotika bei Pilzen versagen, denn bei vielen bekannten Antibiotika sind die Bakterienwand oder die Mitochondrien Hauptangriffspunkte dieser Stoffe.

Es gibt auch einige Antibiotika, die auch systemisch, also nicht nur topisch, gegen Pilzinfektionen eingesetzt werden können und die sich durch eine Polyenstruktur auszeichnen, wie beispielsweise Amphotericin B, Griseofulvin, Natamycin und Nystatin. Synthetische systemische Antimycotica sind Flucytosin und eine Reihe von Azolderivaten wie Ketoconazol und Miconazol oder Fluconazol und Itraconazol.

Während der genaue Wirkungsmechanismus der Polyenantibiotika noch nicht in allen Einzelheiten bekannt ist, wirken die synthetischen Azolderivate auf die Ergostorolsynthese in der Zellmembran und beeinflussen damit die Durchlässigkeit der Zellwand.

Ein Nachteil aller bisher bekannten auch systemisch einsetzbaren Antimycotika besteht darin, daß sie in der Regel nicht fungizid, sondern nur fungistatisch wirken und damit eine längere Behandlungsdauer erforderlich machen. Weiterhin gibt es Kreuzresistenzen sowohl bei den Polyenantibiotika wie auch bei den Azolverbindungen und schließlich kommt hinzu, daß der Preis dieser Produkte relativ hoch ist, was einer breiten Anwendung in der Veterinärmedizin entgegensteht.

Es besteht daher noch ein weiteres Bedürfnis nach Veterinärarzneimitteln, die in der Lage sind, Diarrhoeen bei Tieren, die im wesentlichen durch pathogene Pilze bedingt sind, wirksam bekämpfen zu können.

Völlig überraschend wurde jetzt festgestellt, daß bei Verwendung von Escherichia coli Stamm DSM 6601 derartige Diarrhoeen gut bekämpfbar sind, selbst wenn die sonst übliche Medikation mit pilzwirksamen Verbindungen versagt hat.

Escherichia coli, im folgenden als E. coli abgekürzt, fällt in zahlreichen Varianten an, die sich hinsichtlich der Kapselantigene, Oberflächenantigene und Flagellenantigene unterscheiden und daher in zahlreiche serologische Typen unterteilt werden können. Die Einordnung nach Serotypen besagt allerdings nichts über die unterschiedliche Virulenz der Erreger. Vertreter ein- und desselben Serotyps können sowohl im menschlichen als auch im tierischen Körper ein unterschiedliches Pathogenitätspotential besitzen, das im Extremfall von avirulent bis hochgradig pathogen reichen kann. Es ist allerdings bekannt, daß der E. coli Stamm DSM 6601 nicht als human- oder tierpathogen bewertet wird. Dieser Stamm wird beispielsweise in der Humanmedizin als Substitutionspräparat bei infektiösen Darmerkrankungen durch Salmonellen oder Shigellen eingesetzt, und zwar sowohl bei akuten wie auch bei chronischen Fällen. Auch bei sonstigen Störungen der Darmflora wie z.B. nach Antibiotikabehandlung oder Bestrahlung wird dieser E. coli Stamm mit Erfolg in der Substitutionstherapie benutzt. Ob es sich dabei tatsächlich nur um die Verdrängung der pathogenen Bakterienstämme mit den entsprechenden Varianten von E. coli oder Proteus durch diesen speziellen E.-coli-Stamm handelt, also um eine Reduzierung der Toxine, oder ob die Stoffwechselprodukte des Stammes E. coli DSM 6601 von sich aus eine therapeutische Wirksamkeit entfalten, ist nicht eindeutig geklärt.

Ausgehend von diesen bekannten Erklärungen zum Wirkungsmechanismus von lebenden E.-coli-Keimen war aber in keiner Weise zu erwarten, daß eine Behandlung mit diesen lebenden Kulturen eine überraschend umfassende Wirksamkeit bei der Infektion des Darmtraktes von Tieren mit Pilzen und hier insbesondere Hefen, zeigen würde. Zwar waren in den fünfziger Jahren apathogene E. coli Stämme in der Tiermedizin gelegentlich bei Erkrankungen von Rindern oder Schweinen, die zum Teil auch mit Diarrhoeen einhergingen, eingesetzt worden, aber dabei handelte es sich um die Therapie von Ernährungsstörungen bei Ferkeln (Fischer W., Erfahrungen eines praktischen Tierarztes bei der Behandlung kranker Ferkel in den Jahren 1945 bis 1950: Dissertation, Universität München 1950) oder um die Behandlung der sogenannten Semperkrankheit bei Rindern, der nach den Ergebnissen dieser Veröffentlichung offensichtlich eine Mangelernährung aufgrund der Geologie des Gebietes zugrundelag (Häfele W., Die "Semperkrankheit" eine Ernährungs- und Entwicklungsstörung des Rindes im Hochschwarzwald in der Umgebung von St. Blasien; Dissertation, vet. med. Tierklinik, Universität München 1952). Diese frühen Versuche, bestimmte Coli-Stämme bei speziellen Erkrankungen von Schweinen oder Rindern versuchsweise einzusetzen, waren völlig vereinzelt und haben nicht einmal dazu Veranlassung gegeben, bei bakteriell bedingten Diarrhoeen weitere Versuche solcher Art zu unternehmen. Um so überraschender war die Feststellung, daß der E.-coli-Stamm DSM 6601 eine überraschende Wirkung auch bei Darmerkrankungen zeigt, die ausschließlich oder wesentlich durch Pilze bedingt sind, da die Bekämpfung von Pilzinfektionen, insbesondere wenn die Schleimhäute des Darmes betroffen sind, besonders schwierig ist, da die bei bakteriellen Infektionen eingesetzten Präparate praktisch keine Wirkung zeigen.

Bis heute hin ist über die normale Darmflora bei verschiedenen Tierarten überraschend wenig bekannt, aber ein Befall des Gastrointestinaltraktes mit Pilzen und insbesondere mit Hefen ist immer als pathologisches Geschehen zu deuten.

Die überraschend schnelle Wirksamkeit der Behandlung mit E. coli Stamm DSM 6601 läßt den Schluß zu, daß die Wirkung dieser Behandlung nicht oder nicht nur auf der Substitution der Pilzflora durch eine gesunde Bakterienflora beruht, sondern daß der Stamm in großem Maße zu einer Steigerung der körpereigenen Abwehrkräfte beiträgt, wobei vermutet werden kann daß die Stoffwechselprodukte dieses Stammes eine beträchtliche immunstimulatorische Wirkung haben.

Im folgenden wird die Erfindung anhand eines Beispieles näher erläutert:

### Beispiel:

In einer Milchviehanlage in Sachsen, die im Mittel über einen Tierbestand von 1.480 Milchkühen, 600 Färsen und jungrindern und 185 Kälbern verfügt, war bis Anfang Januar 1997 der Kälberbestand durch sehr gute Aufzuchtergebnisse, geringe Morbidität und Mortalität gekennzeichnet. Manifeste Magen- und Darmerkrankungen mit Durchfällen waren außerordentlich selten. Verluste an Kälbern lagen stets unterhalb der 3 %-Grenze, bezogen auf die Zahl der lebend geborenen Kälber.

Ende Januar 1997 kam es im Bestand plötzlich zum gehäuften Auftreten von Durchfällen mit klinisch markanten Symptomen einer Gastroenteritis. Die Erkrankungen begannen bei den Kälbern im Alter von sieben bis elf Tagen, waren von unterschiedlicher Dauer und durch eine Morbidität von mehr als 90 % und eine Mortalität von mehr als 10 % gekennzeichnet. Dabei war der Krankheitsverlauf durch folgende Symptome geprägt: anfänglich grünlichgelblicher Durchfall mit dickbreiiger Konsistenz, danach wurde der Kot im weiteren Krankheitsverlauf schleimig, zunehmend dünnbreiig und meistens wässrig. Die Werte der Körpertemperaturen waren zu Beginn der Erkrankung leicht erhöht (39,7 bis 39,9° C), sanken dann aber im weiteren Verlauf rasch ab und erreichten nur noch untere Grenzen, teilweise im Bereich von 37,8 bis 37,5° C. Die erkrankten Kälber zeigten ausgeprägte Saugunlust und zunehmende Schwäche, so daß es im weiteren Krankheitsverlauf zum Festliegen kam. Schwerkranke Tiere mußte zwangsernährt werden.

Nach einer Kranheitsdauer von drei bis neun Tagen kam es zu immer höheren Verlusten. Die durchgeführten Sektionen zeigten übereinstimmend höchst auffällige Befunde, nämlich einen flüssigen, flockigen bis leicht blutigen Darminhalt und hochgradige, geschwürige Vormagen- und Labmagenentzündungen. Im Monat April 1997 erreichte die Erkrankungshäufigkeit Werte von nahezu 100 % und die Mortalität stieg auf mehr als 15 %.

Die mikrobiologischen Untersuchungen des Magen- und Darminhaltes sowie der inneren Organe waren anfänglich ohne spezifische Ergebnisse, bis im Februar 1997 bei zwei verendeten Kälbern aus Magen- und Darminhalt Hefen der Gattung Candida (C. glabrata und C. albicans) isoliert wurden. Darmpathogene E. coli wurden nicht gefunden. Der Nachweis dieser Hefen gelang bei allen weiteren Sektionen der verendeten Kälber und bei den Untersuchungen von Kotproben von Kälbern und ihren Müttern. Weiterführende gezielte Untersuchungen von Kot- und Colostrumproben der Kühe und Färsen brachten gleiche Ergebnisse. Ergänzende Untersuchungen auf Viren, nämlich Corona- und Rotaviren, sowie auf Cryptosporidien ergaben nur in zwei Fällen bei verendeten Kälbern Coronaviruspartikel und in einem Fall Rotaviren.

Die Herkunft der Hefen war lange Zeit nicht erklärbar, bis es im März 1997 bei der Untersuchung von als Futtermittel eingesetzten Biertrebern gelang, Hefen (Candida glabrata dominierend) in einem Umfang von 3,6 x 10⁷ KBE/g Futtermittel nachzuweisen.

Die von Anfang an umfangreich durchgeführten therapeutischen Maßnahmen wie Diättränken, Antiphlogistika, Styptica, Elektrolytika und Herz- und Kreislaufmittel führten nur zu unbefriedigenden Ergebnissen. Bei Anwendung von Sulfonamiden und Antibiotika verendeten die Tiere sechs bis acht Stunden nach der Applikation. Nachdem diese Therapien erfolglos blieben, wurden Therapeutika, die Huminsäuren als wirksame Substanzen enthalten, eingesetzt. Auch diese Maßnahmen erbrachten keine erkennbaren Besserungen des Krankheitsgeschehens.

Von den in zwei Monaten vom 24. April bis 23. Juni 1997 lebend geborenen 236 Kälbern erkrankten alle Tiere, wobei 41 Tiere in diesem Zeitraum verendeten, was einem Anteil von 17,4 % entspricht, und zwar 29 Tiere durch Diarrhoeen entsprechend 70,7 % der Verluste und acht Tiere an Diarrhoeen mit begleitender Bronchopneumonie entsprechend 19,5 % der Verluste und vier Tiere aus sonstigen Gründen, entsprechend 9,8 % der Verluste.

Im Zeitraum vom 24. Juni 1997 bis 04. September 1997 wurde dann bei 300 neugeborenen Kälbern eine Suspension von lebenden E. coli Stamm DSM 6601 in einer Menge von täglich 15,0 ml je Kalb gegeben. Diese Dosis war unabhängig vom Gewicht und Alter des Tieres und wurde oral appliziert, und zwar während einer Zeitdauer von 10 bis 13 Tagen post partum. Im Zeitraum vom 24. Juni bis 08. Juli 1997 wurden in dieser Weise 61 Kälber behandelt, von denen zwei an einer Diarrhoe erkrankten, was einer Morbidität von 3,3 % entspricht. Keines der Tiere verendete an dieser Krankheit.

Im Zeitraum vom 09. Juli bis 23. Juli 1997 wurden 49 Kälber entsprechend behandelt, von denen keines an einer Gastroenteritis mit Diarrhoe erkrankte. Die Mortalität an dieser Erkrankung betrug also 0 %.

Im Zeitraum vom 23. Juli bis 06. August 1997 wurden 64 Kälber behandelt, von denen zwei an einer Diarrhoe erkrankten, was einer Morbidität von 3,1 % entspricht. Keines der Tiere verendete an dieser Erkrankung, so daß insoweit die Mortalität gleich 0 ist.

Nachdem alle vorhandenen therapeutischen Möglichkeiten ausgeschöpft waren, konnte durch die prophylaktische und therapeutische Anwendung einer E. coli Stamm DSM 6601-Suspension das Auftreten von Diarrhoeen aufgrund von Gastroenteritiden bei Saugkälbern nahezu vollständig verhindert werden.

Der Einsatz von anderen Arzneimitteln und Diätetika konnte erheblich reduziert werden, so daß die dafür aufzuwendenden Kosten sich um 70 % verringerten.

## Patentansprüche

1. Verwendung von Escherichia coli Stamm DSM 6601 zur Herstellung von Arzneimitteln zur Prophylaxe und Behandlung von im wesentlichen durch pathogene Pilze bedingten Diarrhoeen bei Tieren.

## Claims

1. Use of Escherichia coli strain DSM 6601 for the manufacture of medicaments for prophylaxis and treatment of diarrhoea of animals essentially based on pathogenic fungi.

## Revendications

1. Utilisation d'Escherichia coli souche DSM 6601 pour la manufacture des médicaments pour la prophylaxie et le traitement des diarrhées des animaux essentiellement basées sur des mycophytes pathogènes.
